# EUROPEAN PATENT APPLICATION

(11) **EP 0 620 278 A1**
(43) Date of publication of application: **19.10.1994**
(21) Application number: 94104908.2
(22) Date of filing: 28.03.1994
(51) Int. Cl.: C12N 15/54, C12N 9/10, C12N 1/21, C07K 15/28, A61K 37/52, A61K 39/395

(54) **Human transaldolase and DNA encoding therefor**

(30) Priority: 30.03.1993 JP 71590/93
(71) Applicant: ONO PHARMACEUTICAL CO., LTD., Osaka-shi Osaka (JP)
(72) Inventor: Naito, Takayuki, Ibaraki-shi, Osaka 567 (JP); Nakade, Shinji, c/o Minase Research Institute, Shimamotocho, Mishima-gun, Osaka 618 (JP); Miyamoto, Tsumoru, Shiga-gun, Shiga 520-05 (JP)
(74) Representative: Henkel, Feiler, Hänzel & Partner

(57) **Abstract**

The present invention is related to novel polypeptide consisting of 327 amino acids which is produced in a human glioblastoma cell line, a method of producing it, DNA encoding the said polypeptide, a fragment capable of selectively hybridizing to the DNA sequence, a replication or expression vector comprising the DNA and a host cell tranformed with the replication or expression vector. The polypeptide of the present invention may be useful for the prevention of or in the treatment of aplasia or abnormal proliferation, of glias or neurons, depression or enhancement of activity, or tumors, diabetes mellitus, diseases caused by abnormal glycometabolism or in the treatment diseases related to transaldorase.

## Description

### Field of the Invention

The present invention is related to a novel peptide produced by a certain cell line and DNAs encoding it.

### Purpose of the Invention

Many factors related to maintain, differentiation, proliferation and growth are produced from cells. For example, glia cells which constitute brain as a member, produce many factors related to differentiation, proliferation and growth of neuro-cells and glia cells.

Present inventors have directed their attention to this point and energetic research has been carried out in order to find novel factors (polypeptides) which glia etc. generate.

Until now, when a man skilled in the art intends to obtain a particular polypeptide or a DNA encoding it, he generally utilizes methods by confirming an intended biological activity in a tissue or in a cell medium, isolating and purifying the polypeptide and then cloning a gene or methods by "expression-cloning" with the guidance of the biological activity.

However, physiologically active polypeptides in living body have often many kinds of activities. Therefore, it is increasing that after a gene is cloned, the gene is found to be identical to that encoding a polypeptide already known. Generally glia generates only a very slight amount of a factor and it makes difficult to isolate and to purify the factor and to confirm its biological activity.

On the other hand, preparation technique of cDNA and sequencing technique have been rapidly developed, and it has been able to sequence a large quantity of cDNA. Further, methods of "Reverse Genetics" of characterizing the function of a gene from the sequence of the gene, have been greatly developed.

The present inventors attempted to find novel polypeptides by using these methods. That is, a series of methods was carried out by isolating mRNA from glia etc., obtaining cDNA by using mRNA thus obtained, deciding its nucleotide sequence and deducing its amino acid sequence. In this manner, the present inventors have succeeded to find a quite novel human transaldorase and DNA encoding it, to complete the present invention.

There was no polypeptide having amino acid sequence which is identical to or has high homology to that of the polypeptide of the present invention, when amino acid sequences of the polypeptide was compared by a computer to all known sequences in data base of Swiss Prot (Swiss Prot Release 2.0), except for yeast transaldorase gene.

But, there was no human nucleotide sequence which is identical to or has high homology to that encoding the polypeptide of the present invention, when the DNA sequence was compared by a computer to all known sequences in data base of GenBank (GenBank Release 70.0). Therefore, the polypeptide of the present invention is considered to be quite novel.

We intend to tell about transaldorase herein. Metabolism is an important reaction in living body to maintain life activity of the living body. Glycolytic system is one of the reaction that cells cleave sugar and produce kinds of saccharides which are essential material to biosynthesize energy compounds such as ATP or NADPH etc., nucleic acid or glycoprotein, respectively.

Pentose phosphate pathway is one of the above glycolysis reaction. This pathway supplies NADPH and ribose 5-phosphate etc., which are essential material to biosynthesize fatty acids and nucleic acids.

Transaldorase is an enzyme for this pentose phosphate pathway and catalyze the following reaction.

If transaldorase does not function in the cell, the abnormal metabolism will occurred, and lost the biosynthetic systems will be disordered. It is considered that the lost balance induces hindrance for maintenance or proliferation of the cells. So, transaldorase is an important enzyme to the living organism. 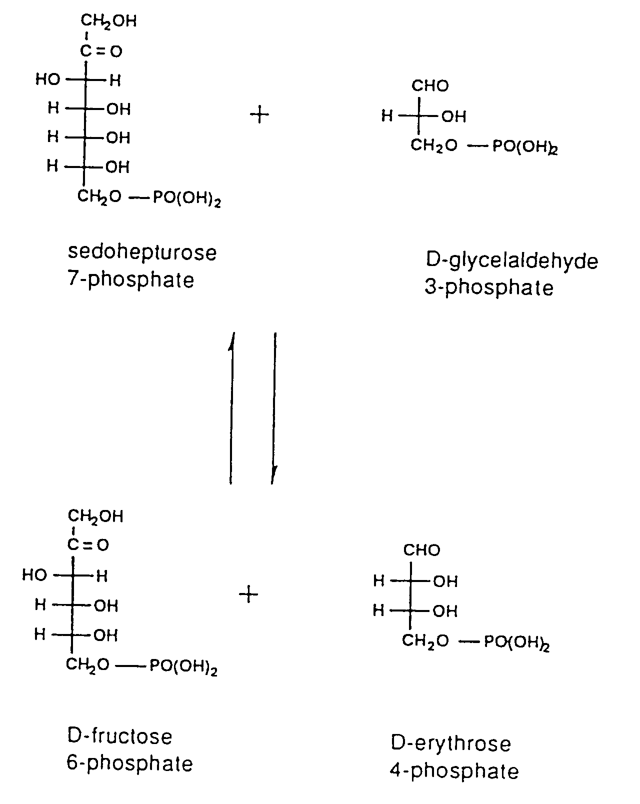
The present invention is concerned with a polypeptide having the amino acid shown in SEQ ID. No. 1, in substantially purified form, a homologue thereof or a fragment of the sequence or homologue of a fragment, and DNA encoding such a polypeptide. More particularly, the present invention is related to DNA having the nucleotide sequence shown in SEQ ID No. 2 or 3, and DNA having a fragment which is selectively hybridizing to nucleotide sequence shown in SEQ ID No. 2 or 3.

The present invention is related to:
(1) a polypeptide having an amino acid sequence shown in SEQ ID NO. 1,
(2) a DNA encoding the polypeptide described above (1),
(3) a DNA having a nucleotide sequence shown in SEQ ID NO. 2, and
(4) a DNA having a nucleotide sequence shown in SEQ ID NO. 3.

A polypeptide of Seq. ID No. 1 in substantially purified form will generally comprise the polypeptide in a preparation in which more than 90%, e.g. 95%, 98% or 99% of the polypeptide in the preparation is that of the Seq. ID No. 1.

A polypeptide homologue of the Seq. ID No. 1 will be generally at least 70%, preferably at least 80 or 90% and more preferably at least 95% homologous to the polypeptide of Seq. ID No. 1 over a region of at least 20, preferably at least 30, for instance 40, 60 or 100 more contiguous amino acids. Such polypeptide homologues will be referred to below as a polypeptide according to the invention.

Generally, fragments of Seq. ID No. 1 or its homologues will be at least 10, preferably at least 15, for example 20, 25, 30, 40, 50 or 60 amino acids in length, and are also encompassed by the term "a polypeptide according to the invention" as used herein.

A DNA capable of selectively hybridizing to the DNA of Seq. ID No. 2 or 3 will be generally at least 70%, preferably at least 80 or 90% and more preferably at least 95% homologous to the DNA of Seq. ID No. 2 or 3 over a region of at least 20, preferably at least 30, for instance 40, 60 or 100 or more contiguous nucleotides. Such DNA will be encompassed by the term "DNA according to the invention".

Fragments of the DNA of Seq. ID No. 2 or 3 will be at least 15, preferably at least 20, for example 25, 30 or 40 nucleotides in length, and are also encompassed by the term "DNA according to the invention" as used herein.

A further embodiment of the invention provides replication and expression vectors comprising DNA according to the invention. The vectors may be, for example, plasmid, virus or phage vectors provided with an origin of replication, optionally a promoter for the expression of the said DNA and optionally a regulator of the promoter. The vector may contain one or more selectable marker genes, for example a anpicillin resistance gene. The vector may be used in vitro, for example of the production of RNA corresponding to the DNA, or used to transfect or transform a host cell.

A further embodiment of the invention provides host cells transformed or transfected with the vectors for the replication and expression of DNA according to the invention, including the DNA SEQ. ID No. 2 or 3 or the open reading frame thereof. The cells will be chosen to be compatible with the vector and may for example be bacterial, yeast, insect or mammalian cells.

A further embodiment of the invention provides a method of producing a polypeptide which comprises culturing host cells of the present invention under conditions effective to express a polypeptide of the invention. Preferably, in addition, such a method is carried out under conditions in which the polypeptide of the invention is expressed and then produced from the host cells.

DNA according to the invention may also be inserted into the vectors described above in an antisense orientation in order to proved for the production of antisense RNA. Antisense RNA may also be produced by synthetic means. Such antisense RNA may be used in a method of controlling the levels of a polypeptide of the invention in a cell.

The invention also provides monoclonal or polyclonal antibodies to a polypeptide according to the invention. The invention further provides a process for the production of monoclonal or polyclonal antibodies to the polypeptides of the invention. Monoclonal antibodies may be prepared by conventional hybridoma technology using a polypeptide of the invention or a fragment thereof, as an immunogen. Polyclonal antibodies may also be prepared by conventional means which comprise inoculating a host animal, for example a rat or a rabbit, with a polypeptide of the invention and recovering immune serum.

The present invention also provides pharmaceutical compositions containing a polypeptide of the invention, or an antibody thereof, in association with a pharmaceutically acceptable diluent and/or carrier.

The polypeptide of the present invention includes that which a part of their amino acid sequence is lacking (e.g., a polypeptide comprised of the only essential sequence for revealing a biological activity in an amino acid sequence shown in SEQ ID No. 1), that which a part of their amino acid sequence is replaced by other amino acids (e.g., those replaced by an amino acid having a similar property) and that which other amino acids are added or inserted into a part of their amino acid sequence, as well as those having the amino acid sequence shown in SEQ ID NO. 1.

As known well, there are one to six kinds of codon as that encoding one amino acid (for example, one kind of codon for Met, and six kinds of codon for leucine (Leu) are known). Accordingly, the nucleotide sequence of DNA can be changed in order to encode the polypeptide having the same amino acid sequence.

The DNA of the present invention, specified in (2) includes a group of every nucleotide sequences encoding polypeptides shown in SEQ ID NO. 1 . There is a probability of improving a yield of production of a polypeptide by changing a nucleotide sequence.

The DNA specified in (3) is the embodiment of DNA shown in (2), and is sequence in the natural form.

The DNA shown in (4) indicates the sequence of the DNA specified in (3) with a non-translational region.

The DNA having a nucleotide sequence shown in SEQ ID NO. 3 may be prepared according to the following methods, that is:
(i) by isolating mRNA from a cell line which produces the polypeptide of the present invention (e.g., human glioblastoma cell line),
(ii) by preparing first strand (single stranded DNA) from mRNA thus obtained, followed by preparing second strand (double stranded DNA) (synthesis of cDNA),
(iii) by inserting cDNA thus obtained into a proper plasmid vector,
(iv) by transforming host cells with the recombinant DNA thus obtained (preparation of cDNA library),
(v) by random-cloning on a large scale from cDNA library thus obtained, followed by sequencing about 300 bases from 5' end of each clone, and
(vi) by sequencing complete length of a clone which has a novel base sequence.

Explained in detail, step (i) may be carried out in accordance with the method of Okayama, H. et al. (described in Methods in Enzymology, vol. 154, p 3, 1987) after a human glioblastoma cell line is stimulated by a proper stimulant (e.g., IL-1 etc.). Examples of the cells which produce the polypeptide of the present invention is preferably human glioblastoma cell line T98G (ATCC strain No., CRL-1690).

Steps (ii), (iii) and (iv) are a series of steps for preparing cDNA library, and may be carried out in accordance with the method of Gubler & Hoffman (Gene, vol. 25, pp 263, 1983) with a slight modification. As examples of the plasmid vector used in the step (iii), many vectors functioning in an E. coli strain (e.g., pBR 322) and in a Bacillus subtilis (e.g., pUB 110) are known, and pVfCS-1 (described in detail in the following Example) prepared from pGEM-3Zf(+) (3,199 bp, manufactured by Promega Corp.) which functions in an E. coli, may be preferably used.

As examples of host used in the step (iv), many cells are already known. Any cells may be used, and DH5 competent cell which has been prepared in accordance with the method described in Gene, vol. 96, pp 23, 1990, may be preferably used.

The cloning in the step (v) may be carried out by methods known per se and the sequencing may be carried out in accordance with the method of Maxam-Gilbert or the dideoxy termination method.

The step (vi) may be carried out in accordance with the method described in Molecular Cloning (written by Sambrook, J., Fritsch, E. F. and Maniatis, T., published by Cold Spring Harbor Laboratory Press in 1989).

As the following step, it is necessary to examine whether or not the DNA thus obtained codes right a secretory protein. The examination requires:
(I) the conversion of the DNA sequence into the amino acid sequence in a possible frame,
(II) the preparation of hydrophobicity profile from the amino acid sequence thus obtained, followed by confirmation of the existence of a highly hydrophobic region just after the translation initiation codon (ATG) (secretory proteins have highly hydrophobic signal peptides on their N-termini), and then
(III) the confirmation that the DNA thus obtained covers complete or almost complete length of intact mRNA. These confirmation may be carried out after the step (vi) hereinbefore described, and effectively between the step (v) and the step (vi).

The Step (III) may be carried out by northern analysis.

Once the nucleotide sequences shown in SEQ ID NOs. 2 and 3 are determined, DNA of the present invention may be obtained by chemical synthesis, by PCR method or by hybridization making use of a fragment of DNA of the present invention, as a probe. Furthermore, DNA of the present invention may be obtained in a desired amount by transforming with a vector DNA inserted a DNA of the present invention into a proper host, followed by culturing the transformant.

The polypeptides of the present invention (shown in SEQ ID NO. 1 ) may be prepared by:
(1) isolating and purifying from an organism or a cultured cell,
(2) chemically synthesizing, or
(3) using a skill of biotechnology,
preferably, by the method described in (3).

Examples of expression system when preparing a polypeptide by using a skill of biotechnology, is, for example, the expression system of bacteria, yeast, insect cell and mammalian cell.

For example, the expression in E. coli may be carried out by adding the initiation codon (ATG) to 5' end of a DNA encoding a nucleotide sequence shown in SEQ ID NO. 3, connecting the DNA thus obtained to the downstream of a proper promoter (e.g., trp promoter, lac promoter, λPL promoter, T7 promoter etc.), and then inserting it into a vector (e.g., pBR322, pUC18, pUC19 etc.) which functions in an E. coli strain to prepare an expression vector.

Then, an E. coli strain (e.g., E. coli DH1 strain, E. coli JM109 strain, E. coli HB101 strain, etc.) which is transformed with the expression vector thus obtained may be cultured in a proper medium to obtain the desired polypeptide. When a signal peptide of bacteria (e.g., signal peptide of pel B) is utilized, the desired polypeptide may be also produced in periplasm. Furthermore, a fusion protein with other polypeptide may be also produced easily.

Furthermore, the expression in a mammalian cell may be carried out, for example, by inserting the DNA shown in SEQ ID NO. 3 into the downstream of a proper promoter (e.g., SV40 promoter, LTR promoter, metallothionein promoter etc.) in a proper vector (e.g., retrovirus vector, papilloma virus vector, vaccinia virus vector, SV40 vector, etc.) to obtain an expression vector, and transforming a proper mammalian cell (e.g., monkey COS-7 cell, Chinese hamster CHO cell, mouse L cell etc.) with the expression vector thus obtained, and then culturing the transformant in a proper medium to get a desired polypeptide in the culture medium. The polypeptide thus obtained may be isolated and purified by conventional biochemical methods.

### Effects of the Invention

Transaldorase of the present invention is produced in a human glioblastoma cell line. Transaldorase is existed as an enzyme of pentose phosphate pathway in glycometabolism. So, it is predicted that transaldorase have activities related to maintenance, proliferation and growth of cells including gas, neurons and other cells widespreadly.

The polypeptide of the present invention may be useful for the prevention of or in the treatment of aplasia or abnormal proliferation of glias or neurons, endothelial cells, epithelial cells, blood cells, muscle cells, bone cells or other types of cells. Otherwise the polypeptide and/or this regulator are also applicable for depression or enhancement of activity of above mentioned cells, or tumors, diabetes mellitus. Diseases caused by abnormal glycometabolism or in the treatment diseases related to transaldorase can be involved in the target for drug development using this invention. Schaaff, I et al (Eur. J. Biochem. 188, 597 (1990)) demonstrated that the transaldorase-deficient strains of the yeast accumulated sedoheptulose 7-phosphate, an intermediate of the pentose-phosphate pathway and this gene is related to cell growth. Therefore, diseases due to accumulation of sedoheptulose 7-phosphate by lack of transaldorase activity can also be target for drug development using this invention.

Further, polyclonal or monoclonal antibody against the polypeptide of the present invention can be used in the determination of the amount of the said polypeptide in organism, and thereby, may be utilized for the purpose of investigating the relationship between the said polypeptide and diseases, or for the purpose of diagnosing diseases, and the like. Polyclonal and monoclonal antibody thereof may be prepared by conventional methods by using the said polypeptide or the fragment thereof as an antigen.

The DNA of the present invention may be utilized as an important and essential template in preparing the polypeptide of the present invention which is expected to possess various use or for diagnosis of and in the treatment of gene diseases (the treatment of gene defect disease and the treatment by inhibiting expression of the polypeptide by antisense DNA (RNA), and the like). Further, genomic DNA may be isolated by using the DNA of the present invention as a probe. Similarly, it is possible to isolate genes having high homology to the DNA of the present invention in human or those of other species.

### Application for Pharmaceuticals

For the purpose of the present invention of or in the treatment of aplasia or abnormal proliferation, of glia or neurons, depression or enhancement of immunological activity, or tumors etc., the polypeptide of the present invention may be normally administered systemically or partially, usually by oral or parenteral administration, preferably orally, intravenously or intraventricularly.

The doses to be administered are determined depending upon age, body weight, symptom, the desired therapeutic effect, the route of administration, and the duration of the treatment etc. In the human adult, the doses per person per dose are generally between 100 µg and 100 mg, by oral administration, up to several times per day, and between 10 µg and 100 mg, by parenteral administration up to several times per day.

As mentioned above, the doses to be used depend upon various conditions. Therefore, there are cases in which doses lower than or greater than the ranges specified above may be used.

Administration of the compounds of the present invention, may be as solid compositions, liquid compositions or other compositions for oral administration, as injections, liniments or suppositories etc. for parenteral administration.

Solid compositions for oral administration include compressed tablets, pills, capsules, dispersible powders, granules. Capsules include soft capsules and hard capsules.

In such compositions, one or more of the active compound(s) is or are admixed with at least one inert diluent (such as lactose, mannitol, glucose, hydroxypropyl cellulose, microcrystalline cellulose, starch, polyvinylpyrrolidone, magnesium metasilicate aluminate, etc.). The compositions may also comprise, as is normal practice, additional substances other than inert diluents: e.g. lubricating agents (such as magnesium stearate etc.), disintegrating agents (such as cellulose calcium glycolate, etc.), stabilizing agents (such as human serum albumin, lactose etc.), and assisting agents for dissolving (such as arginine, asparaginic acid etc.).

The tablets or pills may, if desired, be coated with a film of gastric or enteric material (such as sugar, gelatin, hydroxypropyl cellulose or hydroxypropylmethyl cellulose phthalate, etc.), or be coated with more than two films. And further, coating may include containment within capsules of absorbable materials such as gelatin.

Liquid compositions for oral administration include pharmaceutically-acceptable emulsions, solutions, syrups and elixirs. In such compositions, one or more of the active compound(s) is or are contained in inert diluent(s) commonly used in the art (purified water, ethanol etc.). Besides inert diluents, such compositions may also comprise adjuvants (such as wetting agents, suspending agents, etc.), sweetening agents, flavouring agents, perfuming agents, and preserving agents.

Other compositions for oral administration included spray compositions which may be prepared by known methods and which comprise one or more of the active compound(s). Spray compositions may comprise additional substances other than inert diluents: e.g. stabilizing agents (sodium sulfite etc.), isotonic buffer (sodium chloride, sodium citrate, citric acid, etc.). For preparation of such spray compositions, for example, the method described in the United States Patent No. 2,868,691 or 3,095,355 (herein incorporated in their entireties by reference) may be used.

Injections for parenteral administration include sterile aqueous or non-aqueous solutions, suspensions and emulsions. In such compositions, one or more active compound(s) is or are admixed with at least one inert aqueous diluent(s) (distilled water for injection, physiological salt solution, etc.) or inert non-aqueous diluents(s)(propylene glycol, polyethylene glycol, olive oil, ethanol, POLYSOLBATE 80 TM , etc.).

Injections may comprise additional other than inert diluents: e.g. preserving agents, wetting agents, emulsifying agents, dispersing agents, stabilizing agent (such as human serum albumin, lactose, etc.), and assisting agents such as assisting agents for dissolving (arginine, asparaginic acid, etc.).

They may be sterilized for example, by filtration through a bacteria-retaining filter, by incorporation of sterilizing agents in the compositions or by irradiation. They may also be manufactured in the form of sterile solid compositions, for example, by freeze-drying, and which can be dissolved in sterile water or some other sterile diluents for injection immediately before used.

Other compositions for parenteral administration include liquids for external use, and endermic liniments (ointment, etc.), suppositories for rectal administration and pessaries which comprise one or more of the active compound(s) and may be prepared by known methods.

### Examples

The following examples are illustrated, but not limit, the present invention.

### Example 1: Construction of vector for use in the preparation of cDNA library

A plasmid vector, pGEM-3Zf(+) (3,199 bp, available from Promega Corp.) was digested with HindIII, followed by Klenow treatment and circularized again. The necessary amount of this plasmid was recovered from culture of E. coli transformed with the plasmid. Next, an AatII-NdeI fragment was cut out from the plasmid, and the resulting linear plasmid fragment was smooth-ended using T4 polymerase. After ligating the thus treated termini with a HindIII linker, the resulting fragment was digested with HindIII, circularized again and then transformed into an E. coli strain to recover plasmid.

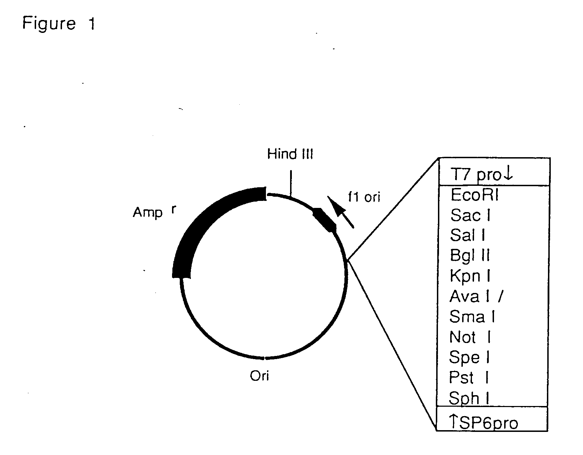

Thereafter, a SacI-PstI portion in the polylinker of the plasmid was replaced by a synthetic polylinker shown below: 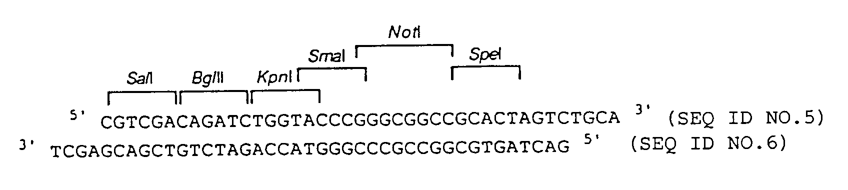
The plasmid vector thus constructed (see Fig. 1) was named pVfCS-1.

The pVfCS-1 has the following characteristic properties as a multi-purpose plasmid vector.
1. Okayama-Berg method and Gubler-Hoffman method can be applied.
2. Plasmid yield per cultured cells is high.
3. Single-stranded DNA can be prepared.
4. A cDNA insert can be cut out easily.
5. Preparation of a deletion mutant for sequencing use can be made easily.
6. In vitro transcription can be made.

### Example 2: Isolation and purification of mRNA

A total of 3 x 10⁷ cells of a human glioblastoma cell line T98G (ATCC strain No., CRL-1690) were stimulated for 4 hours with 100 units/ml of human IL-1β, subsequently isolating mRNA in accordance with the method of Okayama, H. et al. (Methods in Enzymology, vol. 154, pp 3, 1987) as described below.

That is, the stimulated cells were solubilized with a 5.5 M GTC solution (5.5 M guanidine thiocyanate, 25 mM sodium citrate, 0.5% sodium lauryl sarcosine), and the resulting cell lysate was laid on a cushion of a cesium trifluoroacetate (CsTFA) solution having a density of 1.51 and centrifuged (120,000 x g, 20 hours) to recover 1.26 mg of total RNA in the resulting pellet. Thereafter, the RNA sample was passed twice through an oligo(dT)-cellulose column to purify and recover 46 µg of poly(A)⁺RNA.

### Example 3: Preparation of cDNA library

A cDNA library was prepared in accordance with the method of Gubler & Hoffman (Gene, vol. 25, pp 263, 1983) with a slight modification. A first strand was synthesized from the poly(A)⁺ RNA (5 mg) prepared in Example 2, using a reverse transcriptase and an oligo(dT) primer having a NotI site. After synthesizing a second strand and carrying out SalI adapter ligation and NotI digestion, the adapter and the primer were removed by gel filtration column chromatography using a column packed with Sephacryl S-500HR (available from Pharmacia), thereby recovering a fraction containing 820 ng of cDNA. The above cDNA synthesizing step was effected making use of a kit (Super Script System, available from BRL).

Separately from this, a vector was prepared by subjecting the pVfCS-1 obtained in Example 1 to complete digestion with NotI, digesting the product further with SalI, subjecting the resulting digest to 0.8% agarose gel electrophoresis to cut out a band of interest and then purifying the vector of interest making use of a kit for glass powder method use (GENECLEAN II, available from BIO 101).

After subjecting the thus prepared cDNA and vector to ligation, the resulting product was transfected into DH5 competent cells which have been prepared in accordance with the method of Inoue, H. et al. (Gene, vol. 96, pp 23, 1990). As the results, a cDNA library containing 6 x 10⁵ independent clones with an average length of 1.5 kb was obtained.

### Example 4: Cloning and Sequencing

Using the cDNA library prepared in Example 3, clones were plated on LB-broth agar containing ampicillin with a density of 300 colonies/dish having a diameter of 10 cm. Cloning was carried out by picking up the colonies at random. Each of the colonies was cultured overnight in 3 ml of LB-broth. A 200 µl portion of the resulting culture was mixed with dimethylsulfoxide (DMSO, final concentration of 7%) and stored at -80°C, and the remaining portion of the culture was used for the isolation of plasmid. The purification of plasmid was carried out by the usual way.

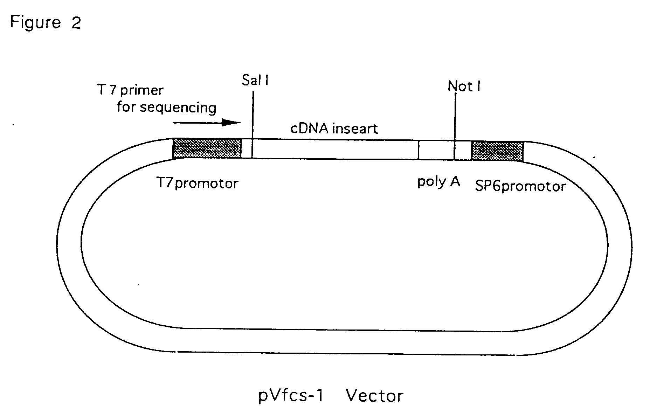

Since the plasmid has a structure shown in Fig. 2, its nucleotide sequence can be read from the 5' end of the cloned cDNA when sequencing is carried out using T7 primer. DNA sequencing was carried out in accordance with a cycle sequence method based on the dideoxy termination method of Sanger, F. et al., using a fluorescence dye terminator of ABI (Applied Biosystems Inc.). Reading of the sequence was carried out using a DNA sequencer of ABI (Model 373A). In this way, a nucleotide sequence having a length of about 300 bases from 5' end of each cDNA was obtained.

### Example 5: Analysis of partial sequence data

Using the FASTA program of Lipman, D. J. and Pearson, W. R., the nucleotide sequence obtained in Example 4 was searched for its homology with every nucleotide sequence contained in known data bases (GenBank and EMBL). As the results, a clone having an unknown sequence was identified. The unknown nucleotide sequence was converted into amino acid sequences in possible three frames. It is possible however that the cloned cDNA does not cover complete length of mRNA. If it does not cover the complete length, it is less possible that the clone contains the N-terminal amino acid sequence moiety.

In consequence, northern analysis was carried out in order to determine if the clone TG1390 has complete length or not. That is, poly(A)⁺RNA which has been extracted and purified from a glioblastoma cell line was subjected to electrophoresis and then blotting on nylon membrane. When hybridization was carried out using a TG1390 cDNA insert as a probe, a single band was found at a position corresponding to about 1000 bp. Since the TG1390 cDNA insert had a size of about 1200 bp, it was confirmed that the TG1390 clone was a almost complete length cDNA.

### Example 6: Determination of complete sequence of the cDNA and open reading frame

The complete length cDNA sequence was determined by means of random sequencing in accordance with the method described in Molecular Cloning (Sambrook, J., Fritsch, E. F. and Maniatis, T., Cold Spring Harbor Laboratory Press, 1989). Plasmid was recovered from the TG1390 clone to isolate and purify a cDNA insert. The insert thus purified was subjected to ligation and fragmentation, followed by smooth-ending of both termini of the resulting DNA fragment with T4 polymerase, thereby recovering a DNA fragment of about 400 bp in length by agarose gel electrophoresis. The thus obtained DNA fragment was cloned into a SmaI site of a plasmid vector BLUESCRIPT II (available from Stratagene) and then transfected into an E. coli strain. A total of 20 colonies were picked up at random to prepare 20 corresponding plasmid DNA samples (every of them contained a TG1390 cDNA fragment as an insert) which were subsequently subjected to DNA sequencing.

DNA sequencing and sequence reading were carried out in the same manner as the procedure described in Example 4. Sequence data of the TG1390 cDNA fragment were arranged into a continued sequence making use of a DNA sequence connection program of DNASIS, thereby obtaining a base sequence shown in SEQ ID NO. 3. An open reading frame was determined based on the complete length cDNA sequence data to deduce corresponding amino acid sequence, with the results shown in SEQ ID NO. 1.

Complete nucleotide sequence of the TG1390 cDNA and primary amino acid sequence of the TG1390 protein encoded by the nucleotide sequence are shown in SEQ ID NO. 4.

SEQUENCE LISTING

(1) GENERAL INFORMATION:

(i) APPLICANT:
(A) NAME: Ono Pharmaceutical Co., Ltd.
(B) STREET: 1-5, Doshomachi 2-chome
(C) CITY: Chuo-ku, Osaka-shi
(D) STATE: Osaka
(E) COUNTRY: Japan
(F) POSTAL CODE (ZIP): 541
(G) TELEPHONE: 06-222-5551
(H) TELEFAX: 06-222-5706

(ii) TITLE OF INVENTION: Novel Polypeptide and DNAs Encoding It

(iii) NUMBER OF SEQUENCES: 6

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 327 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 1:

Met Glu Ser Ala Leu Asp Gln Leu Lys Gln Phe Thr Thr Val Val Ala
1               5                   10                  15

Asp Thr Gly Asp Phe His Ala Ile Asp Glu Tyr Lys Pro Gln Asp Ala
            20                  25                  30

Thr Thr Asn Pro Ser Leu Ile Leu Ala Ala Ala Gln Met Pro Ala Tyr
        35                  40                  45

Gln Glu Leu Val Glu Glu Ala Ile Ala Tyr Gly Arg Lys Leu Gly Gly
    50                  55                  60

Ser Gln Glu Asp Gln Ile Lys Asn Ala Ile Asp Lys Leu Phe Val Leu
65                  70                  75                  80

Phe Gly Ala Glu Ile Leu Lys Lys Ile Pro Gly Arg Val Ser Thr Glu

                85                  90                  95

Val Asp Ala Arg Leu Ser Phe Asp Lys Asp Ala Met Val Ala Arg Ala
            100                 105                 110

Arg Arg Leu Ile Glu Leu Tyr Lys Glu Ala Gly Ile Ser Lys Asp Arg
        115                 120                 125

Ile Leu Ile Lys Leu Ser Ser Thr Trp Glu Gly Ile Gln Ala Gly Lys
    130                 135                 140

Glu Leu Glu Glu Gln His Gly Ile His Cys Asn Met Thr Leu Leu Phe
145                 150                 155                 160

Ser Phe Ala Gln Ala Val Ala Cys Ala Glu Ala Gly Val Thr Leu Ile
                165                 170                 175

Ser Pro Phe Val Gly Arg Ile Leu Asp Trp His Val Ala Asn Thr Asp
            180                 185                 190

Lys Lys Ser Tyr Glu Pro Leu Glu Asp Pro Gly Val Lys Ser Val Thr
        195                 200                 205

Lys Ile Tyr Asn Tyr Tyr Lys Lys Phe Ser Tyr Lys Thr Ile Val Met
    210                 215                 220

Gly Ala Ser Phe Arg Asn Thr Gly Glu Ile Lys Ala Leu Ala Gly Cys
225                 230                 235                 240

Asp Phe Leu Thr Ile Ser Pro Lys Leu Leu Gly Glu Leu Leu Gln Asp
                245                 250                 255

Asn Ala Lys Leu Val Pro Val Leu Ser Ala Lys Ala Ala Gln Ala Ser
            260                 265                 270

Asp Leu Glu Lys Ile His Leu Asp Glu Lys Ser Phe Arg Trp Leu His
        275                 280                 285

Asn Glu Asp Gln Met Ala Val Glu Lys Leu Ser Asp Gly Ile Arg Lys
    290                 295                 300

Phe Ala Ala Asp Ala Val Lys Leu Glu Arg Met Leu Thr Glu Arg Met
305                 310                 315                 320

Phe Asn Ala Glu Asn Gly Lys
                325

(2) INFORMATION FOR SEQ ID NO: 2:

(i) SEQUENCE CHARACTERISTICS:
(A) LENGTH: 981 base pairs
(B) TYPE: nucleic acid
(C) STRANDEDNESS: single
(D) TOPOLOGY: linear

(ii) MOLECULE TYPE: cDNA to mRNA

(xi) SEQUENCE DESCRIPTION: SEQ ID NO: 2:

ATGGAGTCCG CGCTGGACCA GCTCAAGCAG TTCACCACCG TGGTGGCCGA CACGGGCGAC 60

TTCCACGCCA TCGACGAGTA CAAGCCCCAG GATGCTACCA CCAACCCGTC CCTGATCCTG 120

GCCGCAGCAC AGATGCCCGC TTACCAGGAG CTGGTGGAGG AGGCGATTGC CTATGGCCGG 180

AAGCTGGGCG GGTCACAAGA GGACCAGATT AAAAATGCTA TTGATAAACT TTTTGTGTTG 240

TTTGGAGCAG AAATACTAAA GAAGATTCCG GGCCGAGTAT CCACAGAAGT AGACGCAAGG 300

CTCTCCTTTG ATAAAGATGC GATGGTGGCC AGAGCCAGGC GGCTCATCGA GCTCTACAAG 360

GAAGCTGGGA TCAGCAAGGA CCGAATTCTT ATAAAGCTGT CATCAACCTG GGAAGGAATT 420

CAGGCTGGAA AGGAGCTCGA GGAGCAGCAC GGCATCCACT GCAACATGAC GTTACTCTTC 480

TCCTTCGCCC AGGCTGTGGC CTGTGCCGAG GCGGGTGTGA CCCTCATCTC CCCATTTGTT 540

GGGCGCATCC TTGATTGGCA TGTGGCAAAC ACCGACAAGA AATCCTATGA GCCCCTGGAA 600

GACCCTGGGG TAAAGAGTGT CACTAAAATC TACAACTACT ACAAGAAGTT TAGCTACAAA 660

ACCATTGTCA TGGGCGCCTC CTTCCGCAAC ACGGGCGAGA TCAAAGCACT GGCCGGCTGT 720

GACTTCCTCA CCATCTCACC CAAGCTCCTG GGAGAGCTGC TGCAGGACAA CGCCAAGCTG 780

GTGCCTGTGC TCTCAGCCAA GGCGGCCCAA GCCAGTGACC TGGAAAAAAT CCACCTGGAT 840

GAGAAGTCTT TCCGTTGGTT GCACAACGAG GACCAGATGG CTGTGGAGAA GCTCTCTGAC 900

GGGATCCGCA AGTTTGCCGC TGATGCAGTG AAGCTGGAGC GGATGCTGAC AGAACGAATG 960

TTCAATGCAG AGAATGGAAA G 981

(2) INFORMATION FOR SEQ ID NO: 3:

(i) SEQUENCE CHARACTERISTICS:
(A) LENGTH: 1111 base pairs
(B) TYPE: nucleic acid
(C) STRANDEDNESS: single
(D) TOPOLOGY: linear

(ii) MOLECULE TYPE: cDNA to mRNA

(xi) SEQUENCE DESCRIPTION: SEQ ID NO: 3:

CCCACGCGTC CGCCCACGCG TCCGCGTGAA GCGTCAGAGG ATGGAGTCCG CGCTGGACCA 60

GCTCAAGCAG TTCACCACCG TGGTGGCCGA CACGGGCGAC TTCCACGCCA TCGACGAGTA 120

CAAGCCCCAG GATGCTACCA CCAACCCGTC CCTGATCCTG GCCGCAGCAC AGATGCCCGC 180

TTACCAGGAG CTGGTGGAGG AGGCGATTGC CTATGGCCGG AAGCTGGGCG GGTCACAAGA 240

GGACCAGATT AAAAATGCTA TTGATAAACT TTTTGTGTTG TTTGGAGCAG AAATACTAAA 300

GAAGATTCCG GGCCGAGTAT CCACAGAAGT AGACGCAAGG CTCTCCTTTG ATAAAGATGC 360

GATGGTGGCC AGAGCCAGGC GGCTCATCGA GCTCTACAAG GAAGCTGGGA TCAGCAAGGA 420

CCGAATTCTT ATAAAGCTGT CATCAACCTG GGAAGGAATT CAGGCTGGAA AGGAGCTCGA 480

GGAGCAGCAC GGCATCCACT GCAACATGAC GTTACTCTTC TCCTTCGCCC AGGCTGTGGC 540

CTGTGCCGAG GCGGGTGTGA CCCTCATCTC CCCATTTGTT GGGCGCATCC TTGATTGGCA 600

TGTGGCAAAC ACCGACAAGA AATCCTATGA GCCCCTGGAA GACCCTGGGG TAAAGAGTGT 660

CACTAAAATC TACAACTACT ACAAGAAGTT TAGCTACAAA ACCATTGTCA TGGGCGCCTC 720

CTTCCGCAAC ACGGGCGAGA TCAAAGCACT GGCCGGCTGT GACTTCCTCA CCATCTCACC 780

CAAGCTCCTG GGAGAGCTGC TGCAGGACAA CGCCAAGCTG GTGCCTGTGC TCTCAGCCAA 840

GGCGGCCCAA GCCAGTGACC TGGAAAAAAT CCACCTGGAT GAGAAGTCTT TCCGTTGGTT 900

GCACAACGAG GACCAGATGG CTGTGGAGAA GCTCTCTGAC GGGATCCGCA AGTTTGCCGC 960

TGATGCAGTG AAGCTGGAGC GGATGCTGAC AGAACGAATG TTCAATGCAG AGAATGGAAA 1020

GTAGCGCATC CCTGAGGCTG GACTCCAGAT CTGCACCGCC GGCCAGCTGG GATCTGACTG 1080

CACGTGGCTT CTGATGAATC TTGCGTTTTT T 1111

(2) INFORMATION FOR SEQ ID NO: 4:

(i) SEQUENCE CHARACTERISTICS:
(A) LENGTH: 1111 base pairs
(B) TYPE: nucleic acid
(C) STRANDEDNESS: single
(D) TOPOLOGY: linear

(ii) MOLECULE TYPE: cDNA to mRNA

(vi) ORIGINAL SOURCE:
(A) ORGANISM: Homo sapiens
(H) CELL LINE: T98G

(ix) FEATURE:
(A) NAME/KEY: CDS
(B) LOCATION: 41..1021

(xi) SEQUENCE DESCRIPTION: SEQ ID NO: 4:

CCCACGCGTC CGCCCACGCG TCCGCGTGAA GCGTCAGAGG ATG GAG TCC GCG CTG 55

Met Glu Ser Ala Leu
1               5

GAC CAG CTC AAG CAG TTC ACC ACC GTG GTG GCC GAC ACG GGC GAC TTC     103
Asp Gln Leu Lys Gln Phe Thr Thr Val Val Ala Asp Thr Gly Asp Phe
                10                  15                  20

CAC GCC ATC GAC GAG TAC AAG CCC CAG GAT GCT ACC ACC AAC CCG TCC     151
His Ala Ile Asp Glu Tyr Lys Pro Gln Asp Ala Thr Thr Asn Pro Ser
            25                  30                  35

CTG ATC CTG GCC GCA GCA CAG ATG CCC GCT TAC CAG GAG CTG GTG GAG     199
Leu Ile Leu Ala Ala Ala Gln Met Pro Ala Tyr Gln Glu Leu Val Glu
        40                  45                  50

GAG GCG ATT GCC TAT GGC CGG AAG CTG GGC GGG TCA CAA GAG GAC CAG     247
Glu Ala Ile Ala Tyr Gly Arg Lys Leu Gly Gly Ser Gln Glu Asp Gln
    55                  60                  65

ATT AAA AAT GCT ATT GAT AAA CTT TTT GTG TTG TTT GGA GCA GAA ATA     295
Ile Lys Asn Ala Ile Asp Lys Leu Phe Val Leu Phe Gly Ala Glu Ile
70                  75                  80                  85

CTA AAG AAG ATT CCG GGC CGA GTA TCC ACA GAA GTA GAC GCA AGG CTC     343
Leu Lys Lys Ile Pro Gly Arg Val Ser Thr Glu Val Asp Ala Arg Leu
                90                  95                  100

TCC TTT GAT AAA GAT GCG ATG GTG GCC AGA GCC AGG CGG CTC ATC GAG     391
Ser Phe Asp Lys Asp Ala Met Val Ala Arg Ala Arg Arg Leu Ile Glu
            105                 110                 115

CTC TAC AAG GAA GCT GGG ATC AGC AAG GAC CGA ATT CTT ATA AAG CTG     439
Leu Tyr Lys Glu Ala Gly Ile Ser Lys Asp Arg Ile Leu Ile Lys Leu
        120                 125                 130

TCA TCA ACC TGG GAA GGA ATT CAG GCT GGA AAG GAG CTC GAG GAG CAG     487
Ser Ser Thr Trp Glu Gly Ile Gln Ala Gly Lys Glu Leu Glu Glu Gln
    135                 140                 145

CAC GGC ATC CAC TGC AAC ATG ACG TTA CTC TTC TCC TTC GCC CAG GCT     535
His Gly Ile His Cys Asn Met Thr Leu Leu Phe Ser Phe Ala Gln Ala
150                 155                 160                 165

GTG GCC TGT GCC GAG GCG GGT GTG ACC CTC ATC TCC CCA TTT GTT GGG     583
Val Ala Cys Ala Glu Ala Gly Val Thr Leu Ile Ser Pro Phe Val Gly
                170                 175                 180

CGC ATC CTT GAT TGG CAT GTG GCA AAC ACC GAC AAG AAA TCC TAT GAG     631
Arg Ile Leu Asp Trp His Val Ala Asn Thr Asp Lys Lys Ser Tyr Glu
            185                 190                 195

CCC CTG GAA GAC CCT GGG GTA AAG AGT GTC ACT AAA ATC TAC AAC TAC     679
Pro Leu Glu Asp Pro Gly Val Lys Ser Val Thr Lys Ile Tyr Asn Tyr
        200                 205                 210

TAC AAG AAG TTT AGC TAC AAA ACC ATT GTC ATG GGC GCC TCC TTC CGC     727

Tyr Lys Lys Phe Ser Tyr Lys Thr Ile Val Met Gly Ala Ser Phe Arg
    215                 220                 225

AAC ACG GGC GAG ATC AAA GCA CTG GCC GGC TGT GAC TTC CTC ACC ATC 775
Asn Thr Gly Glu Ile Lys Ala Leu Ala Gly Cys Asp Phe Leu Thr Ile
230                 235                 240                 245

TCA CCC AAG CTC CTG GGA GAG CTG CTG CAG GAC AAC GCC AAG CTG GTG 823
Ser Pro Lys Leu Leu Gly Glu Leu Leu Gln Asp Asn Ala Lys Leu Val
                250                 255                 260

CCT GTG CTC TCA GCC AAG GCG GCC CAA GCC AGT GAC CTG GAA AAA ATC 871
Pro Val Leu Ser Ala Lys Ala Ala Gln Ala Ser Asp Leu Glu Lys Ile
            265                 270                 275

CAC CTG GAT GAG AAG TCT TTC CGT TGG TTG CAC AAC GAG GAC CAG ATG 919
His Leu Asp Glu Lys Ser Phe Arg Trp Leu His Asn Glu Asp Gln Met
        280                 285                 290

GCT GTG GAG AAG CTC TCT GAC GGG ATC CGC AAG TTT GCC GCT GAT GCA 967
Ala Val Glu Lys Leu Ser Asp Gly Ile Arg Lys Phe Ala Ala Asp Ala
    295                 300                 305

GTG AAG CTG GAG CGG ATG CTG ACA GAA CGA ATG TTC AAT GCA GAG AAT 1015
Val Lys Leu Glu Arg Met Leu Thr Glu Arg Met Phe Asn Ala Glu Asn
310                 315                 320                 325

GGA AAG TAGCGCATCC CTGAGGCTGG ACTCCAGATC TGCACCGCCG GCCAGCTGGG 1071
Gly Lys

ATCTGACTGC ACGTGGCTTC TGATGAATCT TGCGTTTTTT 1111

(2) INFORMATION FOR SEQ ID NO: 5:

(i) SEQUENCE CHARACTERISTICS:
(A) LENGTH: 41 base pairs
(B) TYPE: nucleic acid
(C) STRANDEDNESS: double
(D) TOPOLOGY: linear

(ii) MOLECULE TYPE: DNA (Synthetic DNA)

(xi) SEQUENCE DESCRIPTION: SEQ ID NO: 5:

CGTCGACAGA TCTGGTACCC GGGCGGCCGC ACTAGTCTGC A 41

(2) INFORMATION FOR SEQ ID NO: 6:

(i) SEQUENCE CHARACTERISTICS:
(A) LENGTH: 41 base pairs
(B) TYPE: nucleic acid
(C) STRANDEDNESS: double

(D) TOPOLOGY: linear
(ii) MOLECULE TYPE: DNA (Synthetic DNA)
(xi) SEQUENCE DESCRIPTION: SEQ ID NO: 6:
GACTAGTGCG GCCGCCCGGG TACCAGATCT GTCGACGAGC T 41

### Brief description of the drawings

Figure 1 shows the construction of plasmid vector, pVfCS-1.

Figure 2 shows the construction of a recombinant DNA into which cDNA derived from human glioblastoma cell line T98G is inserted.

## Claims

1. A polypeptide having the amino acid sequence shown in SEQ ID No. 1 in substantially purified form, a homologue thereof or a fragment of the sequence or homologue of a fragment.

2. A polypeptide according to claim 1 having the amino acid sequence shown in SEQ ID No. 1.

3. DNA encoding a polypeptide according to claim 1.

4. DNA according to claim 3 having the nucleotide sequence shown in SEQ ID No. 2 or a fragment thereof capable of selectively hybridizing to SEQ ID No. 2.

5. DNA according to claim 3 having the nucleotide sequence shown in SEQ ID No. 3 or a fragment thereof capable of selectively hybridizing to SEQ ID No. 3.

6. A replication and expression vector comprising DNA according to any one of claims 3 to 5.

7. Host cells transformed or tranfected with a replication and expression vector according to claim 6.

8. A method of producing a polypeptide which comprises culturing host cells according to claim 7 under conditions effective to express a polypeptide according to claim 1 or 2.

9. A monoclonal or polyclonal antibody to a polypeptide according to claim 1 or 2.

10. A pharmaceutical composition containing a polypeptide according to claims 1 or 2 or an antibody according to claim 9 in association with a pharmaceutically acceptable diluent and/or carrier.
